# FASCICULE DE BREVET EUROPEEN

(11) **EP 0 681 828 B1**
(45) Date de publication et mention de la délivrance du brevet: **27.12.1996**
(21) Numéro de dépôt: 95400575.7
(22) Date de dépôt: 15.03.1995
(51) Int. Cl.: A61K 7/09

(54) **Procédé pour la déformation permanente des matières kératiniques**
Verfahren zur dauerhaften Verformung der keratinischen Substanzen
Process for permanent deformation of keratinous matter

(30) Priorité: 11.04.1994 FR 9404228
(43) Date de publication de la demande: 15.11.1995
(73) Titulaire: L'OREAL, 75008 Paris (FR)
(72) Inventeur: Samain, Henri, F-91570 Bievres (FR)
(74) Mandataire: Tezier Herman, Béatrice

(56) Documents cités:
- EP-A- 0 551 135
- CH-A- 299 882
- CH-A- 373 867
- FR-A- 2 486 395
- GB-A- 1 125 794
- US-A- 4 982 750

## Description

La présente invention concerne un nouveau procédé de traitement des matières kératiniques, en particulier des cheveux, en vue d'obtenir une déformation permanente de ces dernières, en particulier sous la forme de cheveux permanentés, ledit procédé étant notamment utilisable dans le domaine des salons de coiffure, de beauté, de cosmétique et analogues, professionnels.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur (étape de réduction) puis, après avoir de préférence rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites ou des thiols. Parmi ces derniers, on peut citer la cystéïne et ses divers dérivés, la cystéamine et ses dérivés, l'acide thiolactique, l'acide thioglycolique ainsi que ses esters, notamment le monothioglycolate de glycérol, et le thioglycérol. A cet égard, et bien que possédant une odeur désagréable, l'acide thioglycolique est particulièrement efficace, et constitue ainsi le composé de référence en permanente pour réduire les liaisons disulfures de la kératine ; la cystéïne, quant à elle, produit une odeur beaucoup plus faible que celle de l'acide thioglycolique ou du monothioglycolate de glycérol, mais le degré de frisure obtenu est malheureusement inférieur et loin d'être totalement satisfaisant.

Concernant les compositions oxydantes nécessaires à la mise en oeuvre de l'étape de fixation, on fait le plus souvent appel, dans la pratique, à des compositions à base d'eau oxygénée ou de bromates alcalins, ces agents oxydants étant généralement utilisés à des concentrations supérieures à 0, 8 N. Or, il s'avère que l'emploi de tels agents oxydants à de telles concentrations présente notamment pour inconvénient de conduire à une dégradation plus ou moins marquée de la couleur originelle de la chevelure.

En outre, et en particulier dans le cas où l'agent réducteur utilisé est l'acide thioglycolique, on observe que la succession des cycles réduction - oxydation (i.e. d'opérations de permanente) sur le cheveu conduit, de manière néfaste, à une dégradation progressive non seulement de- la couleur de ce dernier (décoloration) mais également de sa tenue mécanique (diminution de l'énergie de rupture) en particulier dûe à une augmentation significative du taux d'acide kératocystéïque dans le cheveu traité.

La présente invention a notamment pour but de résoudre les problèmes ci-dessus.

Plus précisément encore, la présente invention a pour but de proposer un nouveau procédé de traitement convenant à la déformation permanente des matières kératiniques et qui permette de s'affranchir de la mise en oeuvre des étapes classiques de fixation utilisant des agents oxydants puissants en quantité importante.

Elle a également pour but de proposer un procédé tel que ci-dessus qui permette en outre d'obtenir des frisures de haute qualité et présentant une bonne tonicité.

Elle a également pour but de proposer un procédé tel que ci-dessus qui permette de limiter, voire supprimer, la dégradation mécanique du cheveu, après répétition du traitement.

Elle a aussi pour but de proposer un procédé tel que ci-dessus, limitant, voire supprimant, la décoloration du cheveu.

Elle a enfin pour but de proposer un procédé tel que ci-dessus qui soit, globalement, peu odorant d'une part, et peu irritant pour la peau et/ou le cuir chevelu d'autre part.

Or, il a été trouvé par la demanderesse que ces buts, et d'autres, pouvaient être atteints avec succès en procédant à une sélection convenable de la composition dite réductrice de départ associée à un mode opératoire particulier de mise en oeuvre de cette composition. Cette découverte est à la base de la présente invention.

Ainsi, il est maintenant proposé selon la présente invention un nouveau procédé de traitement convenant à la déformation et/ou la mise en forme, et ceci de manière permanente, des matières kératiniques, et en particulier des cheveux, ledit procédé étant caractérisé par le fait qu'il comprend les étapes suivantes :
(i) on applique sur la matière kératinique à traiter une composition réductrice contenant un thiol choisi dans le groupe constitué par la cystéïne, la cystéamine, l'acide thioglycolique, l'acide thiolactique, l'acide 3-mercaptopropionique, leurs sels et les esters desdits acides, les moyens (tels que par exemple rouleaux, bigoudis et analogues) nécessaires à la mise sous tension mécanique de la matière kératinique étant mis en oeuvre avant, pendant ou après ladite application,
(ii) éventuellement, on soumet ensuite la matière kératinique ainsi traitée à un traitement thermique,
(iii) puis on rince la matière kératinique ainsi traitée,
(iv) on laisse ensuite reposer la matière kératinique ainsi rincée,
(v) on applique sur la matière kératinique ainsi reposée une composition dite composition acide" contenant au moins un acide carboxylique,
(vi) on laisse ensuite reposer la matière kératinique ainsi traitée,
(vii) et enfin, on rince à nouveau la matière kératinique ainsi traitée,
la matière kératinique étant séparée des moyens de mise sous tension utilisés à l'étape (i) soit avant ou après l'application de ladite composition acide, soit avant ou après le rinçage selon l'étape (vii).

Le procédé selon l'invention convient particulièrement bien à l'obtention d'une chevelure permanentée.

Appliqué sur une chevelure saine, et même répété plusieurs fois, le procédé selon l'invention présente pour avantages principaux, entre autres, de conduire, et ceci sans dégagement d'odeurs désagréables d'une part et d'une façon non irritante pour la peau et/ou le cuir chevelu d'autre part, à des cheveux non décolorés ou substantiellement non décolorés, résistants mécaniquement, et présentant de belles frisures.

Mais d'autres caractéristiques, aspects et avantages de l'invention apparaîtront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toute matière kératinique en général, notamment cils, moustaches, poils, laine et autres.

Les agents réducteurs utilisés dans le cadre du procédé selon l'invention sont la cystéïne, la cystéamine, l'acide thioglycolique, l'acide thiolactique, l'acide 3-mercaptopropionique, leurs sels et les esters desdits acides.

Parmi les sels acceptables de la cystéïne et de la cystéamine, on peut plus particulièrement citer les chlorhydrates, les bromhydrates, les citrates, les acétates et les sulfates.

Parmi les sels acceptables des acides thioglycolique, thiolactique et 3-mercaptopropionique, on peut plus particulièrement citer les sels d'ammonium, les sels d'amine primaire, secondaire ou tertiaire, les sels de métaux alcalino-terreux. Comme sels d'amine primaire, secondaire ou tertiaire, on peut citer respectivement la monoéthanolamine, la diisopropanolamine et la triéthanolamine.

Parmi les esters desdits acides, on peut citer le monothioglycolate de glycérol, le monothioglycolate d'éthylène glycol, le mélange azéotrope de thioglycolate d'hydroxy-2-propyle et de thioglycolate d'hydroxy-2-méthyl-1 éthyle décrit dans la demande de brevet FR-A-2 679 448, le monothiolactate de glycérol, le monothiolactate d'éthylène glycol, le 3-mercaptopropionate de glycérol, le 3-mercaptopropionate d'éthylène glycol.

Ces agents réducteurs sont généralement mis en oeuvre dans des compositions cosmétiquement acceptables, lesquelles sont par ailleurs déjà bien connues en soi dans l'état de l'art existant des formulations frisantes destinées à réaliser la première étape (réduction) d'une opération de permanente. Ainsi, à titre d'additifs usuels et classiques, utilisables seuls ou en mélanges, on peut plus particulièrement mentionner les agents tensioactifs de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, et de préférence comprise entre 0,5 et 10% en poids, par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les demandes de brevets français n° 2 598 613 et 2 470 596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2 535 730, les polyorganosiloxanes à groupements aminoalkyles modifiés par des groupements alcoxycarbonyalkyles tels que ceux décrits dans le brevet US n° 4 749 732, des polyorganosiloxanes tels que le copolymère polydiméthylsiloxane-polyoxyalkyle du type Diméthicone Copolyol, un polydiméthylsiloxane à groupements terminaux stéaroxy- (stéaroxydiméthicone), un copolymère polydiméthylsiloxane-dialkylammonium acétate ou un copolymère polydiméthyl-siloxane polyalkylbétaïne décrits dans la demande de brevet britannique n° 2 197 352, des polysiloxanes organo modifés par des groupements mercapto ou mercaptoalkyles tels que ceux décrits dans le brevet français n° 1 530 369 et dans la demande de brevet européen n° 295 780, ainsi que des silanes tels que le stéaroxytriméthylsilane.

La composition réductrice peut également contenir d'autres ingrédients traitants tels que des polymères cationiques tels que ceux utilisés dans les compositions des brevets français n° 79.32078 (FR-A- 2 472 382) et 80.26421 (FR-A- 2 495 931), ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO2/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylène glycol, le monométhyléther de dipropylène glycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

Lorsque l'on utilise la cystéïne ou l'un de ses sels, à titre d'agent réducteur, le pH de la composition réductrice est compris entre 7, 5 et 11, 5, de préférence entre 9 et 10.

Lorsque l'on utilise la cystéamine ou l'un de ses sels, à titre d'agent réducteur, le pH de la composition réductrice est compris entre 7 et 11, de préférence entre 8 et 9.

Lorsque l'on utilise l'acide thioglycolique ou l'acide thiolactique ou l'acide 3-mercaptopropionique, ou l'un de leurs sels, à titre d'agent réducteur, le pH de l'ensemble de la composition réductrice est de préférence compris entre 6,5 et 11,5 et encore plus préférentiellement entre 7 et 10.

Lorsque l'on utilise les esters de l'acide thioglycolique ou de l'acide thiolactique ou de l'acide 3-mercaptopropionique, à titre d'agent réducteur, le pH de l'ensemble de la composition réductrice est de préférence compris entre 5 et 11 et encore plus préférentiellement entre 6 et 10.

Ces pH peuvent être obtenus et/ou ajustés classiquement par ajout soit d'agents basifiants, tels que par exemple l'ammoniaque, la monoéthanolamine, la diéthanolamine, la triéthanolamine, l'isopropanolamine, la di-isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

Dans les compositions réductrices de permanente utilisables dans le cadre de l'invention, les agents réducteurs mentionnés ci-avant sont généralement présents à une concentration qui peut être comprise entre 1 et 30 % en poids, et de préférence entre 3 et 20 % en poids par rapport au poids total de la composition réductrice.

La composition réductrice peut se présenter sous la forme d'une lotion, épaissie ou non, d'une crème, d'un gel, ou de toute autre forme appropriée.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol, ou de l'isopropanol ou encore du glycérol à une concentration maximale de 20 % par rapport au poids total de la composition.

Le véhicule des compositions est de préférence l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs utilisés selon l'invention sont associés à au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N, N'-diacétyl-cystamine, la cystine, la pantéthine, et les disulfures des N-(mercapto-alkyl)w-hydroxyalkylamides décrits dans la demande de brevet EP-A-354 835, les disulfures des N-mono ou N,N-dialkylmercapto-4 butyramides décrits dans la demande de brevet EP-A-368 763, les disulfures des aminomercapto-alkylamides décrits dans la demande de brevet EP-A-432 000, les disulfures des dérivés des acides N-(mercaptoalkyl)-succinamiques ou des N-(mercaptoalkyl)-succinimides décrits dans la demande de brevet EP-A-465 342, et les disulfures des alkylaminomercaptoalkylamides décrits dans la demande de brevet EP-A-514 282. Ces disulfures sont généralement présents dans un rapport molaire de 0,5 à 2,5, et de préférence de 1 à 2, par rapport à l'agent réducteur (voir brevet US 3 768 490).

Conformément à la première étape du procédé selon la présente invention (étape (i)), les compositions contenant le ou les agents réducteurs selon l'invention sont alors appliquées sur les cheveux à traiter, lesquels auront été de préférence préalablement mouillés.
Cette application peut être réalisée avant, pendant ou après l'habituelle étape de mise sous tension des cheveux sous une forme correspondant à la forme finale désirée pour ces derniers (boucles par exemple), cette étape pouvant elle-même être mise en oeuvre par tout moyen, mécanique notamment, approprié et connu en soi pour maintenir sous tension des cheveux, tels que par exemple rouleaux, bigoudis et analogues.

Selon une étape facultative du procédé de l'invention, (étape (ii)), on peut, après application de la composition réductrice, soumettre la chevelure à un traitement thermique, c'est-à-dire un chauffage. Dans ce cas, la température de chauffage est généralement comprise entre 30 et 60°C. Bien que non obligatoire, ce chauffage est néanmoins préféré, car il permet d'ajuster à volonté le degré final de frisure du cheveu. Bien entendu, un travail à température ambiante est possible, et n'est donc pas exclu du cadre de l'invention. Dans la pratique, cette opération peut être conduite au moyen d'un casque de coiffure, d'un sèche cheveux, d'un dispensateur de rayons infra-rouges, ou de tout autre appareil chauffant classique.

Avant de procéder à l'étape suivante de rinçage, il convient, de manière classique, de laisser reposer pendant quelques minutes, généralement entre 2 et 30 mn, de préférence entre 5 et 20 mn, la chevelure sur laquelle a été appliquée la composition réductrice, et ceci de façon à bien laisser le temps au réducteur d'agir correctement sur les cheveux; pendant cette phase d'attente, qui intègre l'étape facultative de chauffage mentionnée ci-avant, on prend soin que les cheveux ne sèchent pas complètement et restent ainsi humides jusqu'au moment de la mise en oeuvre de l'étape suivante (utilisation possible de bonnets, de gels de protection par exemple).

Dans une troisième étape indispensable du procédé selon l'invention (étape (iii)), les cheveux imprégnés de composition réductrice sont ensuite rincés soigneusement, généralement à l'eau.

Selon une quatrième étape absolument essentielle du procédé de traitement selon l'invention (étape (iv)), les cheveux ainsi rincés sont ensuite laissés dans une phase de repos ou d'attente. Selon l'invention, cette phase de repos (ou d'attente) des cheveux rincés peut durer entre 3 et 60 min, et elle est de préférence comprise entre 5 et 30 min. Elle est généralement conduite en laissant reposer à l'air libre (température ambiante) les cheveux mouillés issus du rinçage. Une phase d'attente conduite à température plus élevée n'est pas exclue, et convient également; elle peut être conduite jusqu'à séchage total de la matière kératinique. On peut rincer à nouveau les cheveux après cette phase d'attente.

Dans le procédé selon l'invention, lorsque la composition réductrice contient de l'acide thioglycolique, de l'acide thiolactique ou de l'acide 3-mercaptopropionique ou leurs sels à une concentration supérieure à 8 % en poids par rapport au poids total de la composition, le pH de la composition réductrice est de préférence inférieure à 9 et la phase d'attente de l'étape (iv) est inférieure à 7 minutes.

Selon une cinquième étape essentielle du procédé de traitement selon l'invention (étape(v)), on applique sur la matière kératinique une composition, dite "composition acide", contenant au moins un acide carboxylique. On peut séparer, avant ou après ladite application de la dite composition acide, la matière kératinique des moyens de mise sous tension utilisés à l'étape (i).

Par acide carboxylique, on entend désigner et couvrir notamment les acides carboxyliques simples, les acides polycarboxyliques et les acides (poly)hydroxy(poly)carboxyliques, qui peuvent bien entendu être pris seuls ou en mélange.

A titre d'acides carboxyliques utilisables dans les compositions selon l'invention, on peut plus particulièrement citer les acides lactique, tartrique, acétique, glycolique et citrique.

Selon un mode particulièrement préféré de réalisation du procédé de la présente invention, l'acide mis en oeuvre est l'acide citrique.

La concentration en acide carboxylique dans ladite composition acide est généralement comprise entre 0, 2% et 40 % en poids par rapport au poids total de la composition acide, et de préférence comprise entre 1 et 20% en poids. Le pH de la composition dite acide est compris entre 1, 8 et 7, 5 et peut être ajusté à l'aide d'un agent alcalin choisi parmi l'ammoniaque, la monoéthanolamine, la diéthanolamine, la triéthanolamine, la propanediamine-1, 3, le carbamate d'ammonium, un carbonate ou bicarbonate alcalin ou d'ammonium, un carbonate organique, un hydroxyde alcalin. Bien entendu, selon le pH de la composition dite acide, l'acide carboxylique présent peut se trouver sous forme libre (notamment sous forme d'acide) ou lié (notamment sous forme de sel).

Des odeurs rémanentes pouvant se développer sur les cheveux après un tel traitement, la demanderesse a découvert que ces odeurs pouvaient être atténuées, voire supprimer, en appliquant selon le présent procédé une composition acide contenant, outre l'acide carboxylique, un agent oxydant en très faible quantité.

La composition acide peut donc contenir au moins un agent oxydant à une concentration comprise entre 5 10⁻⁵ et 0, 17 **N** et de préférence comprise entre 10⁻⁴ et 0, 17 **N**. L'agent oxydant est choisi notamment parmi l'eau oxygénée, les bromates alcalins, les persels, les polythionates, la chloramine T, le **N**-bromosuccinimide, le **N**-chlorosuccinimide, les dérivés halogénés des hydantoïnes. L'agent oxydant étant utilisé à très faible concentration, I'étape d'application de la composition acide ne peut être considérée comme une étape de fixation.

La composition acide peut contenir des ajuvants cosmétiques tels que ceux cités ci-dessus pour la composition réductrice. La composition acide peut se présenter sous forme de lotion, épaissie ou non, d'une crème, d'un gel, de shampooing, d'après-shampooing, ou de toute autre forme appropriée.

Lorsque la composition acide est appliquée sur les cheveux avant de les séparer des moyens de mise sous tension, on peut laisser reposer la chevelure pendant quelques minutes (par exemple 5 minutes) puis enlever de la chevelure les moyens mécaniques qui maintenaient sous tension et dans la forme désirée les cheveux pendant le traitement.

On peut dans une autre variante du procédé, séparer les cheveux des moyens de mise sous tension puis appliquer la composition acide sur les cheveux et masser les cheveux afin de favoriser l'imprégnation de la composition acide dans les cheveux.

Dans une sixième étape essentielle du procédé selon l'invention (étape (vi)), les cheveux ainsi traités sont ensuite laissés dans une phase de repos ou d'attente. Selon l'invention, cette phase de repos des cheveux traités peut durer entre 10 secondes et 30 mn, et elle est de préférence comprise entre 1 et 15 mn.

Enfin, dans la dernière étape du procédé selon l'invention (étape (vii)), les cheveux imprégnés de la composition acide sont rincés soigneusement, généralement à l'eau. On peut séparer, avant ou après le rinçage, la matière kératinique des moyens de tensions utilisés à l'étape (i) si cette opération n'a pas été effectuée avant ou après l'application de la composition acide.

On obtient finalement une chevelure présentant de belles frisures, les boucles étant toniques, et ceci sans avoir eu à mettre en oeuvre d'étape chimique de fixation (oxydation).

L'invention a également pour objet un dispositif à plusieurs compartiment ou "kit", caractérisé en ce qu'il comprend dans un premier compartiment une composition réductrice telle que définie précédemment et dans un deuxième compartiment une composition dite acide telle que définie précédemment, ledit dispositif étant réalisé en vue de mettre en oeuvre le procédé selon l'invention.

Des exemples concrets illustrant l'invention vont maintenant être donnés. Aux fins d'une comparaison significative, les mêmes cheveux de départ (avant traitement) ont été utilisés pour tous les exemples.

### Exemple 1 (invention)

On utilise une composition réductrice présentant les caractéristiques suivantes :

| | |
|---|---|
| - acide thioglycolique | 9 g |
| - ammmoniaque qs | pH 7 |
| - carbonate d'ammonium | 2 g |
| - monoéthanolamine qs | pH 8,5 |
| - eau déminéralisée qsp | 100 g |

et une composition acide ayant les caractéristiques suivantes :

| | |
|---|---|
| - acide citrique | 10 g |
| - ammoniaque qs | pH 4,5 |
| - chloramine T | 0,2 g soit 0,014 N |
| - eau déminéralisée qsp | 100 g |

Le mode opératoire est le suivant : on applique sur une mèche de cheveux enroulés et humides (diamètre des rouleaux: 9 mm) la composition réductrice ci-dessus; puis on pose un bonnet en plastique sur la mèche (ce qui permet d'éviter le séchage des cheveux pendant l'étape suivante de chauffage) qui est alors installée sous casque (45°C) pendant 15 minutes; puis on enlève le bonnet et on rince abondamment et soigneusement à l'eau; on remet la mèche sous casque (45°C) pendant 15 minutes puis on rince à nouveau à l'eau. On applique ensuite la composition acide ci-desus et on laisse reposer 5 minutes à température ambiante. Puis on rince abondamment à l'eau et on retire le rouleau (déroulage).

On obtient finalement une mèche présentant une belle frisure; les cheveux après un passage de peigne reprennent rapidement leur forme bouclée et présentent peu d'odeur.

### Exemple 2 (comparatif)

On procède à un procédé de déformation permanente utilisant une étape de fixation.

On utilise une composition fixatrice (oxydante) présentant les caractéristiques suivantes :

| | |
|---|---|
| - eau oxygénée à 35 % en poids | 7 g soit 1,44 N |
| - acide citrique qs | pH 3 |
| - eau déminéralisée qsp | 100 g |

Le mode opératoire est le suivant : on applique sur une mèche de cheveux enroulés et humides (diamètre des rouleaux : 9 mm) la composition réductrice de l'exemple 1; puis on pose un bonnet en plastique sur la mèche qui est alors installée sous casque (45°C) pendant 15 minutes; puis on enlève le bonnet et on rince abondamment et soigneusement à l'eau; on applique ensuite la composition fixatrice ci-dessus et on laisse reposer 10 minutes. On rince enfin abondamment à l'eau et on retire les rouleaux.

L'opération est effectuée trois fois de suite.

### Exemple 3 (invention)

On effectue le procédé de l'exemple 1 trois fois de suite.

On détermine le gain en acide kératocystéique des mèches traitées des exemples 2 et 3 par analyse des acides aminés.
On constate que la mèche de l'exemple 2 présente un gain en acide kératocystéïque de + 0, 9 % alors que la mèche de l'exemple 3 selon l'invention présente un gain en acide kératocystéïque de + 0, 1 %, c'est à dire un gain bien inférieur à celui de l'exemple 2.

### Exemple 4 (invention)

On applique sur une mèche de cheveux enroulés et humides (diamètre des rouleaux : 9 mm) la composition réductrice de l'exemple 1; puis on pose un bonnet en plastique sur la mèche (ce qui permet d'éviter le séchage des cheveux pendant l'étape suivante de chauffage) qui est alors installée sous casque (45°C) pendant 10 minutes; puis on enlève le bonnet et on rince abondamment et soigneusement à l'eau; on remet la mèche sous casque (45°C) pendant 10 minutes puis on rince à nouveau à l'eau. On applique ensuite la composition acide de l'exemple 1 et on laisse reposer 5 minutes à température ambiante. Puis on rince abondamment à l'eau et on retire le rouleau (déroulage).

On obtient finalement une mèche présentant une belle frisure; les cheveux après un passage de peigne reprennent rapidement leur forme bouclée et présentent peu d'odeur.

### Exemple 5 (invention)

On applique sur une mèche de cheveux enroulés et humides (diamètre des rouleaux : 9 mm) la composition réductrice de l'exemple 1; puis on pose un bonnet en plastique sur la mèche (ce qui permet d'éviter le séchage des cheveux pendant l'étape suivante de chauffage) qui est alors installée sous casque (45°C) pendant 15 minutes; puis on enlève le bonnet et on rince abondamment et soigneusement à l'eau; on remet la mèche sous casque (45°C) pendant 15 minutes puis on rince à nouveau à l'eau. On applique ensuite la composition acide de l'exemple 1 et on laisse reposer 2 minutes à température ambiante. Puis on retire le rouleau (déroulage), on masse la mèche de cheveux, on laisse reposer 3 minutes eton rince abondamment à l'eau.

On obtient finalement une mèche présentant une belle frisure; les cheveux après un passage de peigne reprennent rapidement leur forme bouclée et présentent peu d'odeur.

### Exemple 6 (invention)

On applique sur une mèche de cheveux enroulés et humides (diamètre des rouleaux : 9 mm) la composition réductrice de l'exemple 1; puis on pose un bonnet en plastique sur la mèche qui est alors installée sous casque (45°C) pendant 15 minutes; puis on enlève le bonnet et on rince abondamment et soigneusement à l'eau; on remet la mèche sous casque (45°C) pendant 15 minutes puis on rince à nouveau à l'eau. On retire le rouleau (déroulage), on applique la composition acide de l'exemple 1 et on masse la mèche de cheveux. On laisse reposer 5 minutes à température ambiante et on rince abondamment à l'eau.

On obtient finalement une mèche présentant une belle frisure; les cheveux après un passage de peigne reprennent rapidement leur forme bouclée et présentent peu d'odeur.

### Exemple 7 (invention)

On utilise une composition réductrice ayant les caractéristiques suivantes :

| | |
|---|---|
| - acide thioglycolique | 11 g |
| - acide 3- mercaptopropionique | 4 g |
| - ammoniaque qs | pH 7 |
| - carbonate d'ammonium | 2 g |
| - monoéthanolamine qs | pH 8,5 |
| - eau déminéralisée qsp | 100 g |

et une composition acide ayant les caractéristiques suivantes :

| | |
|---|---|
| - acide citrique | 10 g |
| - ammoniaque qs | pH 4,5 |
| - eau oxygénée à 35 % en poids | 0,5 g soit 0,1 N |
| - eau déminéralisée qsp | 100 g |

Le mode opératoire est celui de l'exemple 4.

On obtient finalement une mèche présentant une belle frisure; les cheveux après un passage de peigne reprennent rapidement leur forme bouclée et présentent peu d'odeur.

### Exemple 8 (invention)

On utilise une composition réductrice ayant les caractéristiques suivantes :

| | |
|---|---|
| - cystéïne | 11 g |
| - monoéthanolamine qs | pH 9,5 |
| - eau déminéralisée qs | 100 g |

et une composition acide ayant les caractéristiques suivantes :

| | |
|---|---|
| - acide glycolique | 8 g |
| - ammoniaque qs | pH 4 |
| - eau oxygénée à 35% en poids | 0,5 g |
| - eau déminéralisée qs | 100 g |

Le mode opératoire est celui de l'exemple 1.

On obtient finalement une mèche présentant une belle frisure; les cheveux après un passage de peigne reprennent rapidement leur forme bouclée et présentent peu d'odeur.

### Exemple 9 (invention)

On applique sur une mèche de cheveux enroulés et humides (diamètre des rouleaux : 9 mm) la composition réductrice de l'exemple 8; puis on pose un bonnet en plastique sur la mèche qui est alors installée sous casque (45°C) pendant 30 minutes; puis on enlève le bonnet et on rince abondamment et soigneusement à l'eau; . On applique ensuite la composition acide de l'exemple 1 et on laisse reposer 5 minutes à température ambiante. Puis on rince abondamment à l'eau et on retire le rouleau (déroulage).

On obtient finalement une mèche présentant une belle frisure; les cheveux après un passage de peigne reprennent rapidement leur forme bouclée et présentent peu d'odeur.

### Exemple 10 (invention)

On utilise une composition acide sous forme de shampooing ayant les caractéristiques suivantes :

| | |
|---|---|
| - acide citrique | 8 g |
| - ammoniaque qs | pH 4,5 |
| - lauryl éther sulfate de sodium | 0,3 g MA |
| - chloramine T | 0,12 g |
| - eau déminéralisée qs | 100 g |

Le mode opératoire est le suivant : on applique sur une mèche de cheveux enroulés et humides (diamètre des rouleaux : 9 mm) la composition réductrice de l'exemple 1; puis on pose un bonnet en plastique sur la mèche qui est alors installée sous casque (45°C) pendant 15 minutes; puis on enlève le bonnet et on rince abondamment et soigneusement à l'eau; on remet la mèche sous casque (45°C) pendant 15 minutes puis on rince à nouveau à l'eau. On retire le rouleau (déroulage), on applique la composition acide ci-dessus et on masse la mèche de cheveux comme dans le cadre d'un shampooing, pendant 3 minutes. On rince abondamment à l'eau.

On obtient finalement une mèche présentant une belle frisure; les cheveux après un passage de peigne reprennent rapidement leur forme bouclée et présentent peu d'odeur.

### Exemple 11 (invention)

On utilise une composition acide sous forme de shampooing ayant les caractéristiques suivantes :

| | |
|---|---|
| - acide citrique | 8 g |
| - ammoniaque qs | pH 4,5 |
| - lauryl éther sulfate de sodium | 0,4 g MA |
| - tégobétaïne | 0,4 g MA |
| - eau oxygénée à 35% en poids | 0,7 g soit 0,14 N |
| - eau déminéralisée qs | 100 g |

Le mode opératoire est le suivant : on applique sur une mèche de cheveux enroulés et humides (diamètre des rouleaux : 9 mm) la composition réductrice de l'exemple 1; puis on pose un bonnet en plastique sur la mèche qui est alors installée sous casque (45°C) pendant 15 minutes; puis on enlève le bonnet et on rince abondamment et soigneusement à l'eau; on remet la mèche sous casque (45°C) pendant 15 minutes puis on rince à nouveau à l'eau. On applique la composition acide ci-dessus, on laisse reposer 4 minutes et on retire le rouleau (déroulage). On masse ensuite la mèche de cheveux comme dans le cadre d'un shampooing, pendant 3 minutes, puis on rince abondamment à l'eau.

On obtient finalement une mèche présentant une belle frisure; les cheveux après un passage de peigne reprennent rapidement leur forme bouclée et présentent peu d'odeur.

### Exemple 12 (invention)

On utilise une composition acide ayant les caractéristiques suivantes :

| | |
|---|---|
| - acide citrique | 10 g |
| - ammoniaque qs | pH 4,5 |
| - lauryl éther sulfate de sodium | 0,4 g MA |
| - eau déminéralisée qsp | 100 g |

Le mode opératoire est le suivant : on applique sur les cheveux préalablement enroulés (diamètre des rouleaux : 9 mm) d'une femme japonaise la composition réductrice de l'exemple 7; puis on pose un bonnet en plastique sur les cheveux et on laisse poser sous casque (45°C) pendant 15 minutes. On enlève le bonnet, on rince les cheveux, on replace le bonnet et on laisse poser sous casque (45°C) pendant 15 minutes. On rince à nouveau les cheveux puis on applique la composition acide ci-dessus. On laisse poser 4 minutes à température ambiante et on rince. On retire les rouleaux.

On obtient finalement une belle frisure; les cheveux reprennent après passage du peigne une forme bouclée et présentent peu d'odeur.

## Revendications

1. Procédé de traitement pour la déformation permanente des matières kératiniques, caractérisé en ce qu'il comprend les étapes suivantes :
(i) on applique sur la matière kératinique à traiter une composition réductrice contenant un thiol choisi dans le groupe constitué par la cystéïne, la cystéamine, l'acide thioglycolique, l'acide thiolactique, l'acide 3-mercaptopropionique, leurs sels et les esters desdits acides, les moyens nécessaires à la mise sous tension mécanique de la matière kératinique étant mis en oeuvre avant, pendant ou après ladite application,
(ii) éventuellement, on soumet ensuite la matière kératinique ainsi traitée à un traitement thermique,
(iii) puis on rince la matière kératinique ainsi traitée,
(iv) on laisse ensuite reposer la matière kératinique ainsi rincée,
(v) on applique sur la matière kératinique une composition contenant au moins un acide carboxylique (composition acide),
(vi) on laisse reposer la matière kératinique ainsi traitée,
(vii) et enfin on rince à nouveau la matière kératinique ainsi traitée,
la matière kératinique étant séparée des moyens de mise sous tension utilisés à l'étape (i) soit avant ou après l'application de ladite composition acide (étape (v)), soit avant ou après le rinçage selon l'étape (vii).

2. Procédé selon la revendication 1, caractérisé en ce que l'on utilise la cystéïne et/ou l'un de ses sels, le pH de ladite composition réductrice étant alors compris entre 7,5 et 11,5.

3. Procédé selon la revendication 2, caractérisé en ce que ledit pH est compris entre 9 et 10.

4. Procédé selon la revendication 1, caractérisé en ce que l'on utilise de la cystéamine et/ou l'un de ses sels, le pH de ladite composition réductrice étant alors compris entre 7 et 11.

5. Procédé selon la revendication 4, caractérisé en ce que ledit pH est compris entre 8 et 9.

6. Procédé selon la revendication 1, caractérisé en ce que l'on utilise l'acide thioglycolique ou de l'acide thiolactique ou de l'acide mercaptopropionique ou un de leurs sels, le pH de ladite composition réductrice étant alors compris entre 6 et 11, 5.

7. Procédé selon la revendication 6, caractérisé en ce que ledit pH est compris entre 7 et 10.

8. Procédé selon la revendication 1, caractérisé en ce que l'on utilise un ester de l'acide thioglycolique ou de l'acide thiolactique ou de l'acide 3-mercaptopropionique, le pH de la dite composition étant alors compris entre 5 et 11.

9. Procédé selon la revendication 8, caractérisé en ce que ledit pH est compris entre 6 et 10.

10. Procédé selon l'une des revendications 1 à 5, caractérisé en ce que lorsque le thiol est la cystéïne ou la cystéamine, lesdits sels sont choisis, seuls ou en mélange, parmi les chlorhydrates, les bromhydrates, les citrates, les acétates et les sulfates.

11. Procédé selon l'une des revendications 1 et 6 à 9, caractérisé en ce que lorsque le thiol est choisi parmi l'acide thioglycolique, l'acide thiolactique et l'acide mercaptopropionique, lesdits sels sonts choisis, seuls ou en mélange, parmi les sels d'ammonium, les sels d'amines secondaire ou tertiaire, les sels alcalino-terreux.

12. Procédé selon l'une quelconque des revendications précédentes, caractérisé en ce que le thiol est présent dans ladite composition réductrice dans une teneur allant de 1 à 30% en poids par rapport au poids total de la composition.

13. Procédé selon la revendication 12, caractérisé en ce que ladite teneur est comprise entre 3 et 20% en poids par rapport au poids total de la composition.

14. Procédé selon l'une quelconque des revendications précédentes, caractérisé en ce que , avant ladite application de ladite composition réductrice, la matière kératinique est humidifiée.

15. Procédé selon l'une quelconque des revendications précédentes, caractérisé en ce que ledit traitement thermique (étape (ii)) est conduit à une température comprise entre 30 et 60°C.

16. Procédé selon l'une quelconque des revendications précédentes, caractérisé en ce que , avant de procéder au rinçage (étape(iii)), on laisse reposer (phase d'attente) la matière kératinique issue de l'étape (i), cette phase d'attente incluant l'éventuelle étape (ii).

17. Procédé selon l'une quelconque des revendications précédentes, caractérisé en ce que ladite phase d'attente dure entre 2 et 30 min, de préférence entre 5 et 20 min.

18. Procédé selon l'une quelconque des revendications précédentes, caractérisé en ce que la phase de repos de l'étape (iv) se fait sous chauffage.

19. Procédé selon l'une quelconque des revendications précédentes, caractérisé en ce que la phase de repos de l'étape (iv) dure entre 3 et 60 mn, de préférence entre 5 et 30 mn.

20. Procédé selon l'une quelconque des revendications précédentes, caractérisé en ce que l'étape (iv) est conduite jusqu'à séchage total de la matière kératinique.

21. Procédé selon l'une quelconque des revendications précédentes, caractérisé en ce qu'une nouvelle opération de rinçage est conduite à l'issue de l'étape (iv).

22. Procédé selon l'une quelconque des revendications précédentes, caractérisé en ce que ledit acide carboxylique est choisi, seul ou en mélange, parmi les acides carboxyliques simples, les acides polycarboxyliques, les acides (poly)hydroxy(poly)carboxyliques.

23. Procédé selon la revendication 22, caractérisé en ce que ledit acide carboxylique est choisi, seul ou en mélange, parmi les acides lactique, tartrique, acétique, glycolique et citrique.

24. Procédé selon la revendication 23, caractérisé en ce que ledit acide carboxylique est l'acide citrique.

25. Procédé selon l'une quelconque des revendications 22 à 24, caractérisé en ce que la concentration en acide carboxylique dans ladite composition acide est comprise entre 0, 2 % et 40 %, de préférence entre 1 et 20 % en poids par rapport au poids total de ladite composition acide.

26. Procédé selon l'une quelconque des revendications 22 à 25, caractérisé en ce que le pH de ladite composition acide est compris entre 1,8 et 7,5.

27. Procédé selon la revendication 26, caractérisé en ce que le pH est ajusté à l'aide d'un agent alcalin choisi parmi l'ammoniaque, la monoéthanolamine, la diéthanolamine, la triéthanolamine, la propanediamine-1, 3, le carbamate d'ammonium, un carbonate ou bicarbonate alcalin ou d'ammonium, un carbonate organique, un hydroxyde alcalin.

28. Procédé selon l'une quelconque des revendications 1 et 22 à 27, caractérisé en ce que ladite composition acide contient un agent oxydant à une concentration comprise entre 5 10⁻⁵ et 0, 17 N, de préférence entre 10⁻⁴ et 0, 17 N.

29. Procédé selon la revendication 28, caractérisé en ce que l'agent oxydant est choisi parmi l'eau oxygénée, les bromates alcalins, les persels, les polythionates, la chloramine T, le N-bromosuccinimide, le N-chlorosuccinimide, les dérivés halogénés des hydantoïnes.

30. Procédé selon l'une quelconque des revendications précédentes, caractérisé en ce que ladite composition réductrice et ladite composition acide contiennent des adjuvants destinés à les rendre cosmétiquement acceptables.

31. Procédé selon la revendication précédente, caractérisé en ce que lesdits adjuvants sont choisis, seuls ou en mélange, parmi des agents tensio-actifs de type non-ionique, anionique, cationique ou amphotère, des agents traitants, des ingrédients actifs, des agents antichutes, des agents anti-pelliculaires, des épaississants, des agents de suspension, des agents sequestrants, des agents opacifiants, des colorants, des filtres solaires, des parfums et des conservateurs.

32. Procédé selon l'une quelconque des revendications précédentes, caractérisé en ce que ladite composition réductrice et ladite composition acide se présentent, indépendemment l'une de l'autre, sous la forme d'une lotion, épaissie ou non, d'une crème ou d'un gel.

33. Procédé selon l'une quelconque des revendications précédentes, caractérisé en ce que ladite composition acide se présente sous la forme de shampooing ou d'après shampooing.

34. Procédé selon l'une quelconque des revendications précédentes, caractérisé en ce que la phase de repos de l'étape (vi) dure entre 10 secondes et 30 mn, de préférence entre 1 et 15 mn.

35. Procédé selon l'une quelconque des revendications précédentes, caractérisé en ce que ladite matière kératinique consiste en des cheveux.

36. Dispositif à plusieurs compartiments ou "kit", caractérisé en ce qu'il comprend dans un premier compartiment une composition réductrice telle que définie à l'une quelconque des revendications 1 à 13 et 30 à 32, et, dans un deuxième compartiment, une composition acide telle que définie à l'une quelconque des revendications 1 et 22 à 33.

## Claims

1. Treatment process for the permanent deformation of keratinous matter, characterized in that it comprises the following stages:
(i) a reducing composition containing a thiol chosen from the group consisting of cysteine, cysteamine, thioglycolic acid, thiolactic acid, 3-mercaptopropionic acid, their salts and the esters of the said acids is applied on the keratinous matter to be treated, the means necessary for placing the keratinous matter under mechanical tension being used before, during or after the said application,
(ii) the keratinous matter thus treated is then optionally subjected to a heat treatment,
(iii) the keratinous matter thus treated is then rinsed,
(iv) the keratinous matter thus rinsed is then allowed to rest,
(v) a composition containing at least one carboxylic acid (acidic composition) is applied on the keratinous matter,
(vi) the keratinous matter thus treated is allowed to rest,
(vii) and, finally, the keratinous matter thus treated is rinsed again,
the keratinous matter being separated from the tensioning means used in Stage (i), either before or after the application of the said acidic composition (Stage (v)) or before or after the rinsing according to Stage (vii).

2. Process according to Claim 1, characterized in that cysteine and/or one of its salts is used, the pH of the said reducing composition then being between 7.5 and 11.5.

3. Process according to Claim 2, characterized in that the said pH is between 9 and 10.

4. Process according to Claim 1, characterized in that cysteamine and/or one of its salts is used, the pH of the said reducing composition then being between 7 and 11.

5. Process according to Claim 4, characterized in that the said pH is between 8 and 9.

6. Process according to Claim 1, characterized in that thioglycolic acid or thiolactic acid or mercaptopropionic acid or one of their salts is used, the pH of the said reducing composition then being between 6 and 11.5.

7. Process according to claim 6, characterized in that the said pH is between 7 and 10.

8. Process according to Claim 1, characterized in that an ester of thioglycolic acid or of thiolactic acid or of 3-mercaptopropionic acid is used, the pH of the said composition then being between 5 and 11.

9. Process according to Claim 8, characterized in that the said pH is between 6 and 10.

10. Process according to one of claims 1 to 5, characterized in that, when the thiol is cysteine or cysteamine, the said salts are chosen, alone or as a mixture, from hydrochlorides, hydrobromides citrates, acetates and sulphates.

11. Process according to one of Claims 1 and 6 to 9, characterized in that, when the thiol is chosen from thioglycolic acid, thiolactic acid and mercaptopropionic acid, the said salts are chosen, alone or as a mixture, from ammonium salts, salts of secondary or tertiary amines or alkaline-earth metal salts.

12. Process according to any one of the preceding claims, characterized in that the thiol is present in the said reducing composition in a content ranging from 1 to 30 % by weight with respect to the total weight of the composition.

13. Process according to Claim 12, characterized in that the said content is between 3 and 20 % by weight with respect to the total weight of the composition.

14. Process according to any one of the preceding claims, characterized in that, before the said application of the said reducing composition, the keratinous matter is made wet.

15. Process according to any one of the preceding claims, characterized in that the said heat treatment (Stage (ii)) is carried out at a temperature of between 30 and 60°C.

16. Process according to any one of the preceding claims, characterized in that, before carrying out the rinsing (Stage (iii)), the keratinous matter resulting from Stage (i) is allowed to rest (waiting phase), this waiting phase including the optional Stage (ii).

17. Process according to any one of the preceding claims, characterized in that the said waiting phase lasts between 2 and 30 min and preferably between 5 and 20 min.

18. Process according to any one of the preceding claims, characterized in that the resting phase of Stage (iv) is carried out under heating.

19. Process according to any one of the preceding claims, characterized in that the resting phase of Stage (iv) lasts between 3 and 60 min and preferably between 5 and 30 min.

20. Process according to any one of the preceding claims, characterized in that Stage (iv) is carried cut until the keratinous matter is completely dry.

21. Process according to any one of the preceding claims, characterized in that a new rinsing operation is carried out on conclusion of Stage (iv).

22. Process according to any one of the preceding claims, characterized in that the said carboxylic acid is chosen, alone or as a mixture, from simple carboxylic acids, polycarboxylic acids or (poly)hydroxy(poly) carboxylic acids.

23. Process according to claim 22, characterized in that the said carboxylic acid is chosen, alone or as a mixture, from lactic, tartaric, acetic, glycolic acid citric acids.

24. Process according to Claim 23, characterized in that the said carboxylic acid is citric acid.

25. Process according to any one of Claims 22 to 24, characterized in that the concentration of carboxylic acid in the said acidic composition is between 0.2 % and 40 % and preferably between 1 and 20 % by weight with respect to the total weight of the said acidic composition.

26. Process according to any one of Claims 22 to 25, characterized in that the pH of the said acidic composition is between 1.8 and 7.5.

27. Process according to Claim 26, characterized in that the pH is adjusted using an alkaline agent chosen from ammonia, monoethanolamine, diethanolamine, triethanolamine, 1,3-propanediamine, ammonium carbonate, an alkaline or ammonium carbonate or bicarbonate, an organic carbonate or an alkaline hydroxide.

28. Process according to any one of Claims 1 and 22 to 27, characterized in that the said acidic composition contains an oxidizing agent at a concentration of between 5 × 10⁻⁵ and 0.17 N and preferably between 10⁻⁴ and 0.17 N.

29. Process according to Claim 28, characterized in that the oxidizing agent is chosen from hydrogen peroxide, alkaline bromates, persalts, polythionates, chloramine-T, N-bromosuccinimide, N-chlorosuccinimide or halogenated hydantoin derivatives.

30. Process according to any one of the preceding claims, characterized in that the said reducing composition and the said acidic composition contain adjuvants intended to make them cosmetically acceptable.

31. Process according to the preceding claim, characterized in that the said adjuvants are chosen, alone or as a mixture, from surface-active agents of non-ionic, anionic, cationic or amphoteric type, treatment agents, active ingredients, anti-hair-loss agents, anti-dandruff agents, thickening agents, suspending agents, sequestering agents, opacifying agents, dyes, sunscreens, fragrances and preserving agents.

32. Process according to any one of the preceding claims, characterized in that the said reducing composition and the said acidic composition are provided, independently of one another, in the form of a lotion, which is or is not thickened, of a cream or of a gel.

33. Process according to any one of the preceding claims, characterized in that the said acidic composition is provided in the form of a shampoo or of a conditioner.

34. Process according to any one of the preceding claims, characterized in that the resting phase of Stage (vi) lasts between 10 seconds and 30 min and preferably between 1 and 15 min.

35. Process according to any one of the preceding claims, characterized in that the said keratinous matter consists of hair.

36. Multi-compartment device or "kit", characterized in that it comprises, in a first compartment, a reducing composition as defined in any one of Claims 1 to 13 and 30 to 32 and, in a second compartment, an acidic composition as defined in any one of Claims 1 and 22 to 33.

## Patentansprüche

1. Behandlungsverfahren für die dauerhafte Verformung keratinischer Materialien, das durch die folgenden Schritte gekennzeichnet ist:
(i) Auftragen einer reduzierenden Zusammensetzung, die ein Thiol enthält, das unter Cystein, Cysteamin, Thioglykolsäure, Thiomilchsäure, 3-Mercaptopropionsäure und den Salzen und Estern dieser Säuren ausgewählt ist, auf das zu behandelnde keratinische Material, wobei die Mittel, die erforderlich sind, um das keratinische Material unter mechanische Spannung zu setzen, vor, während oder nach dem Auftragen der reduzierenden Zusammensetzung angebracht werden,
(ii) gegebenenfalls Durchführen einer thermischen Behandlung an dem so behandelten keratinischen Material,
(iii) anschließend Spülen des so behandelten keratinischen Materials,
(iv) dann Ruhenlassen des so gespülten keratinischen Materials,
(v) Auftragen einer Zusammensetzung, die mindestens eine Carbonsäure enthält (saure Zusammensetzung) auf das keratinische Material,
(vi) Ruhenlassen des so behandelten keratinischen Materials, und
(vii) nochmals Spülen des so behandelten keratinischen Materials,
wobei das keratinische Material entweder vor oder nach der Anwendung der sauren Zusammensetzung (Schritt (v)) oder vor oder nach dem Spülen gemäß Schritt (vii) von den in Schritt (i) verwendeten Mitteln, mit denen es unter Spannung gesetzt wird, getrennt wird.

2. Verfahren nach Anspruch 1, dadurch gekennzeichnet, daß Cystein und/oder eines seiner Salze verwendet wird, wobei der pH-Wert der reduzierenden Zusammensetzung dann im Bereich von 7,5 bis 11,5 liegt.

3. Verfahren nach Anspruch 2, dadurch gekennzeichnet, daß der pH-Wert im Bereich von 9 bis 10 liegt.

4. Verfahren nach Anspruch 1, dadurch gekennzeichnet, daß Cysteamin und/oder eines seiner Salze verwendet wird, wobei der pH-wert der reduzierenden Zusammensetzung dann im Bereich von 7 bis 11 liegt.

5. Verfahren nach Anspruch 4, dadurch gekennzeichnet, daß der pH-Wert im Bereich von 8 bis 9 liegt.

6. Verfahren nach Anspruch 1, dadurch gekennzeichnet, daß Thioglykolsäure oder Thiomilchsäure oder Mercaptopropionsäure oder eines ihrer Salze verwendet wird, wobei der pH-Wert der reduzierenden Zusammensetzung dann im Bereich von 6 bis 11,5 liegt.

7. Verfahren nach Anspruch 6, dadurch gekennzeichnet, daß der pH-Wert im Bereich von 7 bis 10 liegt.

8. Verfahren nach Anspruch 1, dadurch gekennzeichnet, daß ein Ester von Thioglykolsäure oder Thiomilchsäure oder 3-Mercaptopropionsäure verwendet wird, wobei der pH-Wert der Zusammensetzung dann im Bereich von 5 bis 11 liegt.

9. Verfahren nach Anspruch 8, dadurch gekennzeichnet, daß der pH-Wert im Bereich von 6 bis 10 liegt.

10. Verfahren nach einem der Ansprüche 1 bis 5, dadurch gekennzeichnet, daß, wenn das Thiol Cystein oder Cysteamin ist, die Salze alleine oder im Gemisch unter Hydrochloriden, Hydrobromiden, Citraten, Acetaten und Sulfaten ausgewählt sind.

11. Verfahren nach einem der Ansprüche 1 und 6 bis 9, dadurch gekennzeichnet, daß, wenn das Thiol unter Thioglykolsäure, Thiomilchsäure und Mercaptopropionsäure ausgewählt ist, die Salze alleine oder im Gemisch unter Ammoniumsalzen, Salzen von sekundären oder tertiären Aminen und Erdalkalisalzen ausgewählt sind.

12. Verfahren nach einem der vorhergehenden Ansprüche, dadurch gekennzeichnet, daß das Thiol in der reduzierenden Zusammensetzung in einem Anteil von 1 bis 30 Gew.-%, bezogen auf das Gesamtgewicht der Zusammensetzung, vorliegt.

13. Verfahren nach Anspruch 12, dadurch gekennzeichnet, daß der Anteil im Bereich von 3 bis 20 Gew.-%, bezogen auf das Gesamtgewicht der Zusammensetzung, liegt.

14. Verfahren nach einem der vorhergehenden Ansprüche, dadurch gekennzeichnet, daß das keratinische Material vor der Anwendung der reduzierenden Zusammensetzung befeuchtet wird.

15. Verfahren nach einem der vorhergehenden Ansprüche, dadurch gekennzeichnet, daß die thermische Behandlung (Schritt (ii)) bei einer Temperatur im Bereich von 30 bis 60 °C durchgeführt wird.

16. Verfahren nach einem der vorhergehenden Ansprüche, dadurch gekennzeichnet, daß das keratinische Material nach Schritt (i) und vor der Durchführung des Spülens (Schritt (iii)) ruhengelassen wird (Wartephase), wobei die Wartephase den gegebenenfalls durchgeführten Schritt (ii) umfaßt.

17. Verfahren nach einem der vorhergehenden Ansprüche, dadurch gekennzeichnet, daß die Wartephase 2 bis 30 min, vorzugsweise 5 bis 20 min dauert.

18. Verfahren nach einem der vorhergehenden Ansprüche, dadurch gekennzeichnet, daß die Ruhephase des Schritts (iv) unter Erwärmen durchgeführt wird.

19. Verfahren nach einem der vorhergehenden Ansprüche, dadurch gekennzeichnet, daß die Ruhephase des Schritts (iv) 3 bis 60 min und vorzugsweise 5 bis 30 min dauert.

20. Verfahren nach einem der vorhergehenden Ansprüche, dadurch gekennzeichnet, daß der Schritt (iv) bis zum vollständigen Trocknen des keratinischen Materials durchgeführt wird.

21. Verfahren nach einem der vorhergehenden Ansprüche, dadurch gekennzeichnet, daß nach Schritt (iv) nochmals gespült wird.

22. Verfahren nach einem der vorhergehenden Ansprüche, dadurch gekennzeichnet, daß die Carbonsäure alleine oder im Gemisch unter einfachen Carbonsäuren, Polycarbonsäuren und (Poly)hydroxy(poly)carbonsäuren ausgewählt ist.

23. Verfahren nach Anspruch 22, dadurch gekennzeichnet, daß die Carbonsäure alleine oder im Gemisch unter Milchsäure, Weinsäure, Essigsäure, Glykolsäure und Citronensäure ausgewählt ist.

24. Verfahren nach Anspruch 23, dadurch gekennzeichnet, daß die Carbonsäure Citronensäure ist.

25. Verfahren nach einem der Ansprüche 22 bis 24, dadurch gekennzeichnet, daß die Konzentration der Carbonsäure in der sauren Zusammensetzung im Bereich von 0,2 bis 40 Gew.-% und vorzugsweise 1 bis 20 Gew.-%, bezogen auf das Gesamtgewicht der sauren Zusammensetzung, liegt.

26. Verfahren nach einem der Ansprüche 22 bis 25, dadurch gekennzeichnet, daß der pH-Wert der sauren Zusammensetzung im Bereich von 1,8 bis 7,5 liegt.

27. Verfahren nach Anspruch 26, dadurch gekennzeichnet, daß der pH-Wert mit einem Mittel zum Alkalischmachen eingestellt wird, das unter Ammoniak, Monoethanolamin, Diethanolamin, Triethanolamin, 1,3-Propandiamin, Ammoniumcarbamat, Alkali- oder Ammoniumcarbonat, Alkali- oder Ammoniumhydrogencarbonat, organischen Carbonaten und Alkalihydroxiden ausgewählt ist.

28. Verfahren nach einem der Ansprüche 1 und 22 bis 27, dadurch gekennzeichnet, daß die saure Zusammensetzung ein Oxidationsmittel in einer Konzentration im Bereich von 5·10⁻⁵ bis 0,17 N und vorzugsweise 10⁻⁴ bis 0,17 N enthält.

29. Verfahren nach Anspruch 28, dadurch gekennzeichnet, daß das Oxidationsmittel unter Wasserstoffperoxid, Alkalibromaten, Persalzen, Polythionaten, Chloramin T, N-Bromsuccinimid, N-Chlorsuccinimid und halogenierten Derivaten von Hydantoinen ausgewählt ist.

30. Verfahren nach einem der vorhergehenden Ansprüche, dadurch gekennzeichnet, daß die reduzierende Zusammensetzung und die saure Zusammensetzung Hilfsstoffe enthalten, die dazu bestimmt sind, sie kosmetisch akzeptabel zu machen.

31. Verfahren nach Anspruch 30, dadurch gekennzeichnet, daß die Hilfsstoffe alleine oder im Gemisch unter nichtionischen, anionischen, kationischen oder amphoteren grenzflächenaktiven Stoffen, Behandlungsmitteln, Wirkstoffen, Mitteln gegen Schuppen, Mitteln gegen Haarausfall, Verdickungsmitteln, Suspensionsmitteln, Maskierungsmitteln, Trübungsmitteln, Färbemitteln, Sonnenschutzfiltern, Parfums und Konservierungsmitteln ausgewählt sind.

32. Verfahren nach einem der vorhergehenden Ansprüche, dadurch gekennzeichnet, daß die reduzierende Zusammensetzung und die saure Zusammensetzung unabhängig voneinander als eingedickte oder nicht eingedickte Lotion, als Creme oder als Gel vorliegen.

33. Verfahren nach einem der vorhergehenden Ansprüche, dadurch gekennzeichnet, daß die saure Zusammensetzung in Form eines Haarwaschmittels oder Haarbehandlungsmittels vorliegt.

34. Verfahren nach einem der vorhergehenden Ansprüche, dadurch gekennzeichnet, daß die Ruhephase des Schritts (vi) 10 Sekunden bis 30 min und vorzugsweise 1 bis 15 min dauert.

35. Verfahren nach einem der vorhergehenden Ansprüche, dadurch gekennzeichnet, daß das keratinische Material aus Haar besteht.

36. Vorrichtung mit mehreren Abteilungen oder "Kit", dadurch gekennzeichnet, daß sie in einer ersten Abteilung eine reduzierende Zusammensetzung nach einem der Ansprüche 1 bis 13 und 30 bis 32, und in einer zweiten Abteilung eine saure Zusammensetzung nach einem der Ansprüche 1 und 22 bis 33 enthält.
